(19) Europäisches Patentamt
European Patent Office
Office européen des brevets

(11) **EP 2 128 815 B1**

(12) **EUROPÄISCHE PATENTSCHRIFT**

(45) Veröffentlichungstag und Bekanntmachung des Hinweises auf die Patenterteilung:
**12.10.2011 Patentblatt 2011/41**

(51) Int Cl.:
***G06T 7/00*** *(2006.01)*

(21) Anmeldenummer: **08155784.5**

(22) Anmeldetag: **07.05.2008**

(54) **Verfahren zur Analyse der Wirkung einer Testsubstanz auf biologische und/ oder biochemische Proben**

Analysis method for analysing the effectiveness of a test substance on biological and/or biochemical samples

Procédé d'analyse de l'effet d'une substance test sur des échantillons biologiques et/ou biochimiques

(84) Benannte Vertragsstaaten:
**CH DE FR GB LI**

(43) Veröffentlichungstag der Anmeldung:
**02.12.2009 Patentblatt 2009/49**

(73) Patentinhaber: **PerkinElmer Cellular Technologies Germany GmbH**
**22525 Hamburg (DE)**

(72) Erfinder:
• **Trede, Dennis**
**28213 Bremen (DE)**
• **Prof. Dr. Maaß, Peter**
**28211 Bremen (DE)**
• **Dr. Preckel, Hartwig**
**21029 Hamburg (DE)**

(74) Vertreter: **von Kreisler Selting Werner**
**Deichmannhaus am Dom**
**Bahnhofsvorplatz 1**
**50667 Köln (DE)**

(56) Entgegenhaltungen:
**WO-A-2006/063216**

• **WU W ET AL: "Experimental designs for optimisation of the image analysis process for cDNA microarrays" CHEMOMETRICS AND INTELLIGENT LABORATORY SYSTEMS, ELSEVIER SCIENCE PUBLISHERS B.V. AMSTERDAM, NL, Bd. 76, Nr. 2, 28. April 2005 (2005-04-28), Seiten 175-184, XP004807699 ISSN: 0169-7439**
• **ZHANG J-H ET AL: "A SIMPLE STATISTICAL PARAMETER FOR USE IN EVALUATION AND VALIDATION OF HIGH THROUGHPUT SCREENING ASSAYS" JOURNAL OF BIOMOLECULAR SCREENING, LARCHMONT, NY, US, Bd. 4, Nr. 2, 1. April 1999 (1999-04-01), Seiten 67-73, XP001145867 ISSN: 1087-0571**
• **BARLOW R; BLAKE J: "Hill coefficients and the logistic equation" TRENDS IN PHARMACOLOGICAL SCIENCES, Bd. 10, Nr. 11, November 1989 (1989-11), Seiten 440-441, XP002502487**

**Beschreibung**

Die Erfindung betrifft ein Verfahren zur Analyse der Wirkung einer Testsubstanz auf biologische und/ oder biochemische Proben, insbesondere auf Proben mit lebenden Zellen. Das erfindungsgemäße Verfahren ist insbesondere auch zur Charakterisierung von Testsubstanzen im HTS oder HCS geeignet.

Das Hochdurchsatz- (HTS) und High-Content-Screening (HCS) sind Methoden der Pharma- und Biotechindustrie, um neue Wirkstoffe gegen schwer therapierbare Krankheiten zu finden. Es werden hierbei biochemische und/ oder biologische Proben, die insbesondere lebende Zellen aufweisen mit Testsubstanzen, wie synthetischen Substanzen in unterschiedlichen Dosierungen behandelt und die Wechselwirkung analysiert. "Hochdurchsatz-Screening" beschreibt hier den sehr hohen Datendurchsatz des Verfahrens (Test von mehr als 100.000 Substanzen pro Tag) und "High-Content-Screening" steht für die hohe insbesondere zelluläre Auflösung der zu analysierenden Signale.

Die Analyse der Wirkung einer Testsubstanz auf eine insbesondere zelluläre Probe erfolgt, indem zunächst eine Mehrzahl von Proben bereitgestellt wird. Die Proben weisen jeweils eine bekannte Konzentration der Testsubstanz auf. Anschließend erfolgt das Durchführen von Messungen, insbesondere im Hochdurchsatz-Screening, wobei die Proben beispielsweise mit sichtbarem oder nicht sichtbarem Licht definierter Wellenlängen bzw. Wellenlängenbereichen beleuchtet werden und insbesondere aufgrund von Lumineszenz von der Probe abgegebene Strahlung detektiert wird. Hierdurch wird eine Mehrzahl von Rohdaten je Probe erfasst. Anschließend erfolgt sodann üblicherweise eine Segmentierung der erhaltenen Rohdaten mit Hilfe von bekannten Bildverarbeitungsroutinen. Hierbei erfolgt insbesondere eine Segmentierung der Zellen und/ oder subzellulärer Strukturen. Für die zu untersuchenden Wechselwirkungen erfolgt sodann das Bereitstellen einer Auswertevorschrift. Durch die Auswertevorschrift bzw. eine Modellierung wird aus den Rohdaten der einzelnen Probe jeweils ein Aktivitätswert ermittelt. Durch den Aktivitätswert wird die Wirkung der Testsubstanz bei der vorliegenden Konzentration beschrieben. Die bereitgestellte Auswertevorschrift wird von mindestens einem Steuer- oder Modellparameter beeinflusst. Es ist somit erforderlich, den mindestens einen Steuerparameter einzustellen. Da die Einstellung der entsprechenden Steuerparameter äußerst komplex ist, kann dies bei Analyseverfahren nach dem Stand der Technik nur durch einen Spezialisten erfolgen, da die Einstellungen der Steuerparameter im Wesentlichen auch auf Erfahrungswerten und biologischer Argumentation basieren. Mit Hilfe der so definierten Auswertevorschrift werden die Bilddaten ausgewertet und zu Dosen $d_i$, $i = 1, ..., n$, ergeben sich Maße $m_i$, die die Wechselwirkung im Bild i quantisieren. Im nächsten Schritt werden diese Daten $(d_i, \ m_i)_{i=1}^{n}$ statistisch ausgewertet.

Zur statistischen Auswertung der Daten gehört z. B. die Berechnung des Z'-Wertes: Ein statistisches Kennzeichen für Hochdurchsatz-Screening, das die Qualität bewertet. Ein weiteres statistisches Merkmal ist die Dosis-/Wirkungs-Kurve, die den Zusammenhang zwischen Substanzaktivität und der Dosis des Wirkstoffs darstellt.

Die Qualität der Analyse ist häufig von geeignet zu wählenden Steuerparametern abhängig. Solche Steuerparameter können z. B. Schwellenwerte oder statistische Größen des Modells sein. Es ist üblich, diese von Hand aufgrund von Erfahrungswerten einzustellen. Weiter besitzt die Anmelderin einen Parameterscanner, der in einer anzugebenden endlichen diskreten Teilmenge aller möglichen Steuerparameter nach einer guten Einstellung sucht. Die Suche nach geeigneten Steuerparametern ist sehr aufwändig.

Aus WO 2006/063216 ist es bekannt Parameter der Bildverarbeitungsroutinen, die zur Auswertung der beim HTS oder HCS aufgenommenen Bilder benutzt werden, automatisch zu optimieren.

Aufgabe der Erfindung ist es, bei einem Verfahren zur Analyse der Wirkung einer Testsubstanz auf biologisch und/ oder biochemische Proben zur Verbesserung der Analyseergebnisse die Einstellung der Steuerparameter zu verbessern. Insbesondere ist es eine weitere Aufgabe der Erfindung, ein derartiges Verfahren zu schaffen, bei dem eine automatische, vorzugsweise optimierte Einstellung der Steuerparameter erfolgt.

Die Lösung der Aufgabe erfolgt durch ein Verfahren zur Analyse der Wirkung einer Testsubstanz auf biologische und/ oder biochemische Proben, die insbesondere lebende Zellen aufweisen, wobei das Verfahren folgende Schritte umfasst:

a. Bereitstellen einer Mehrzahl von Proben im Hochdurchsatz- oder High-Content-Screening, die jeweils eine bekannte Konzentration der Testsubstanz aufweisen, wobei mindestens drei unterschiedliche Konzentrationen der Testsubstanz vorgesehen sind,

b. Durchführen von Bildaufnahmen, die eine Mehrzahl von Bilddaten je Probe liefern;

c. Bereitstellen einer Bildverarbeitungsroutine, die aus den Bilddaten einer Probe jeweils einen Aktivitätswert ermittelt, der die Wirkung der Testsubstanz bei der vorliegenden Konzentration beschreibt und wobei die Auswertevorschrift von mindestens einem Steuerparameter beeinflusst wird;

d. Festlegen mindestens eines Startwerts für den mindestens einen Steuerparameter;

e. Auswerten der Bilddaten mittels der Auswertevorschrift;

f. Bestimmen eines Maßes für die Übereinstimmung der ermittelten Aktivitäten mit einem sich aus theoretischen Überlegungen ergebenden funktionalen Modell mit einer die Abhängigkeit der Aktivitäten von der Konzentration beschreibenden Dosis-/Wirkungskurve;

g. Modifizieren mindestens eines Steuerparameters und Wiederholen der Schritte e. bis g., solange ein Abbruchkriterium nicht erreicht ist.

Dem erfindungsgemäßen Analyseverfahren liegt somit die Erkenntnis einer Parameteroptimierung zugrunde, die insbesondere für die High-Content-Analyse beim Hochdurchsatz-Screeing geeignet ist. Hierbei erfolgt erfindungsgemäß ein Einstellen der Steuerparameter mit Hilfe der insbesondere statistischen Auswertung der Rohdaten mittels der Auswertevorschriften. Durch ein derartiges Rückführen erfolgt iterativ ein Verbessern. Erfindungsgemäß ist dies vorzugsweise möglich, ohne dass die Parameter aufgrund biologischer Begründungen voreingestellt wurden. Die Einstellung muss somit erfindungsgemäß nicht unbedingt durch einen Spezialisten mit entsprechenden Detailkenntnissen erfolgen. Erfindungsgemäß erfolgt somit die Einstellung der Steuerparameter mit Hilfe der Ergebnisse der insbesondere statistischen Auswertung der Rohdaten iterativ. Aufgrund der Iteration kann eine Verbesserung der vorhandenen Steuerparameter bzw. der zur Analyse verwendeten Steuerparameter erfolgen.

Bei den Rohdaten, aus denen die Aktivitätswerte ermittelt werden, handelt es sich insbesondere um Bilddaten. Vorzugsweise umfassen die Bilddaten auch mikroskopische Bilddaten. Insbesondere erfolgt die Erzeugung der Bilddaten durch Beleuchten bzw. Bestrahlen der Probe, wodurch insbesondere ein Anregen der Probe zur Lumineszenz, insbesondere Fluoreszenz erfolgt. Hierzu ist die Probe und/ oder die Testsubstanz vorzugsweise mit Farbstoffmarkern oder dgl. markiert. Besonders bevorzugt ist es hierbei, dass mehrere Messungen je Probe durchgeführt werden. Beispielsweise erfolgt die Messung bei mehreren unterschiedlichen Anregungs- und/ oder Emissionswellenlängen. Ferner können unterschiedliche Polarisationen in der Anregungsstrahlung vorgesehen sein.

Besonders bevorzugt ist es, an einer Probe eine Mehrzahl von Messungen in unterschiedlichen Objektebenen und/ oder zu mehreren aufeinanderfolgenden Zeitpunkten aufzunehmen, um Informationen über die dreidimensionale Struktur und/ oder zeitliche Veränderungen der Probe zu erhalten. In diesem Fall ist es insbesondere bevorzugt, jeweils die aus einer Mehrzahl von Messungen je Probe gewonnen Rohdaten gemeinsam mittels der Auswertevorschrift auszuwerten, um einen Aktivitätswert zu erhalten. Auf diese Weise kann die Aktivität der Testsubstanz aus dem zeitlichen Verlauf derer Wirkung auf die Probe und/ oder aus deren Einfluss auf die dreidimensionale Struktur der Probe ermittelt werden.

Erfindungsgemäß erfolgt ein Bereitstellen einer Mehrzahl von Proben. Dies kann, wie im Hochdurchsatz-Screening üblich, durch das Bereitstellen der Proben in Mikrotiterplatten erfolgen. Vorzugsweise weisen die Proben zwischen drei und dreißig, insbesondere zwischen fünf und fünfzehn unterschiedliche Konzentrationen auf. Die einzelnen Proben weisen somit vorzugsweise die selbe Konzentration an zu untersuchender Probensubstanz, insbesondere dieselbe Menge an Zellsuspension auf, wobei sodann unterschiedliche Konzentrationen an Testsubstanz zugeführt werden. Hierbei ist es wiederum bevorzugt, dass mehrere Proben mit gleicher Konzentration an Testsubstanz bereitgestellt werden. Diese Proben repräsentieren somit den gleichen Punkt der Dosis-/Wirkungs-Kurve. Hierdurch ist es möglich, mit Hilfe statistischer Mittelwerte Einflüsse, beispielsweise von Fehlmessungen oder Fehlkonzentrationen zu verringern.

Vorzugsweise weist jede Probe beispielsweise einer Mikrotiterplatte dieselbe Konzentration an Probensubstanz auf. Dies wird dadurch erzielt, dass beispielweise eine Zellsuspension in gleicher Menge in jedes Well dispensiert wird. Mit hoher Wahrscheinlichkeit kann hierbei davon ausgegangen werden, dass in jedem Well dieselbe Anzahl bzw. dieselbe Größenordnung an Zellen vorhanden ist.

Bei einer bevorzugten Weiterentwicklung des Analyseverfahrens erfolgt insbesondere je Probe die Messung jeweils mehrerer räumlicher Teilbereiche.

Vorzugsweise umfasst die Auswertevorschrift Schritte zur Segmentierung, Objekterkennung, Bestimmung von geometrischen Größen, Helligkeitswerten, Texturparametern von Gesamtbild und/ oder im Bild erkannten Segmenten/ Objekten. Anzahl von Objekten, relativer Position von Objekten zu einander (z. B. Spots in Zellen), Relationen von Helligkeiten in unterschiedlichen Messkanälen (verschiedener Marker), statistischen Größen, die aus den vorher genannten Größen abgeleitet sind, z. B. Quantile, Prozentsatz von Objekten/ Zellen mit einer bestimmten Eigenschaft, Eigenschaften von Untermengen (Teilpopulationen) von Objekten etc.

Die die Auswertevorschrift beeinflussenden Steuerparameter umfassen vorzugsweise mindestens ein oder mehrere der folgenden Steuerparameter: Helligkeitsschwellen, Größenschwellen, Lage und Größe von Masken zur Integration oder Objektsuche sowie Schwellwerte und/ oder Gewichtungsfaktoren für vorstehend genannte Größen oder Kombinationen der Größen.

Vorzugsweise weist das funktionale Modell, das sich aus theoretischen Überlegungen ergibt, für die Dosis-/Wirkungs-Kurve die Hill-Equation auf.

Ein wesentlicher Aspekt des erfindungsgemäßen Analyseverfahrens besteht in dem Bestimmen des Maßes der Übereinstimmung der ermittelten Aktivitäten mit dem funktionalen Modell für die Dosis-/Wirkungs-Kurve. Vorzugsweise erfolgt ein numerisches Anpassen des funktionalen Modells an die ermittelten Aktivitäten. Ferner ist es bevorzugt, eine normierte mittlere quadratische Abweichung der ermittelten Aktivitäten von dem angepassten funktionalen Modell zu bestimmen. Hierdurch ist es durch Durchführung der erfindungsgemäßen Iterationsschritte möglich, gute, insbesondere optimale Steuerparameter auszuwählen.

Vorzugsweise wird das Iterationsverfahren in Abhängigkeit eines Abbruchkriteriums abgebrochen. Hierbei kann es sich beispielsweise um ein Erreichen einer Maximalanzahl an Iterationsschritten handeln. Vorzugsweise dient als Abbruchkriterium eines oder mehrere der nachfolgenden Kriterien:

- Unterschreiten einer vorher bestimmten normierten mittleren quadratischen Abweichung, und/ oder
- Unterschreiten einer vorher bestimmten Mindest-Änderung von Schritt zu Schritt und/ oder
- Erreichen einer Maximalzahl von Iterationsschritten.

Ferner ist es bevorzugt, das Verfahren mehrstufig auszugestalten. Hierbei wird zunächst eine erste Gruppe von Steuerparametern mit Hilfe des erfindungsgemäßen Verfahrens verbessert bzw. optimiert. Dies folgt bis zum Erreichen eines Abbruchkriteriums. Sodann erfolgt ein Verbessern/ Optimieren einer zweiten Gruppe von Steuerparametern. Dies erfolgt bis zum Erreichen eines zweiten Abbruchkriteriums. Gegebenenfalls können auch noch weitere, d. h. dritte, vierte, etc. Gruppen von Steuerparametern vorgesehen und erfindungsgemäß verbessert/ optimiert werden.

Das erfindungsgemäße Analyseverfahren weist insbesondere den Vorteil auf, dass zur Anwendung des Verfahrens kein Spezialwissen oder der Einsatz von besonders geschultem Personal zur Anpassung der Steuerparameter erforderlich ist. Das Analyseverfahren ist somit insbesondere in der bevorzugten Ausführungsform Benutzer unabhängig. Aufgrund der erfolgten erfindungsgemäß möglichen Automatisierung der Auswahl der Steuerparameter kann eine erhebliche Zeitersparnis erzielt werden.

Das erfindungsgemäße Analyseverfahren verwendet die Daten der Dosis-/Wirkungs-Kurve einer Testsubstanz zur Parameteranpassung. Die Abweichung der ermittelten Aktivitäten mit einem sich aus theoretischen Überlegungen ergebenden funktionalem Modell mit einer die Abhängigkeit der Aktivitäten von der Konzentration beschreibenden Dosis-/Wirkungs-Kurve ist während des Durchführens der Optimierungsschritte minimiert. Sobald das Dosis-/Wirkungs-Verhalten auf die Testsubstanz erfindungsgemäß optimiert ist, können mit Hilfe der Analyse gute Ergebnisse für einen großen Bereich an Variationen und für andere Impounds genutzt werden.

Nachfolgend wird die erfindungsgemäße Optimierung der Steuerparameter anhand eines Beispiels näher erläutert. Hierbei zeigen:

Figur 1      eine schematische Darstellung des Ablaufs der einzelnen Verfahrensschritte,

Figur 2      ein Diagramm einer Normierung der Dosis-/Wirkungs-Kurve,

Figur 3      Exemplarische Histogramme der Membranmasken, (a) stimulierte Zelle, (b) unstimulierte Zelle,

Figur 4      Exemplarische Histogramme der Zytoplasmamasken, (a) stimulierte Zelle, (b) unstimulierte Zelle,

Figur 5      Zusammenhang der Quantile $q_1$ und $q_2$ und dem $Z'$-Wert, Akt3- Assay, Whatman-Datensatz,

Figur 6      Entwicklung Z'Wert, Akt3-Assay, Whatman-Datensatz,

Figur 7      Kontroll-Well-Maße nach $Z'$-Optimierung, Akt3-Assay, Whatman- Datensatz,

Figur 8      $\hat{S}$-optimierte Parameter, Akt3-Assay, Whatman-Datensatz,

Figur 9      Entwicklung des $\hat{S}$-Wertes, Akt3-Assay, Whatman-Datensatz,

Figur 10      Ergebnisse der $\hat{S}$-Optimierung, (a) & (b) ursprüngliche Dosis- /Wirkungs-Kurven, (c) & (d) $\hat{S}$-optimierte Kurven, Akt3-Assay, Whatman-Datensatz,

Figur 11      Kontroll-Well-Maße nach $Z'$-Optimierung, Akt3-Assay, PV- Datensatz,

Figur 12    Entwicklung $Z'$-Wert, Akt3-Assay, PV-Datensatz,

Figur 13    Tabelle der $\hat{S}$-optimierten Parameter, Akt3-Assay, PV-Datensatz,

Figur 14    Entwicklung des $\hat{S}$-Wertes, Akt3-Assay, PV-Datensatz,

Figur 15    Ergebnisse der $\hat{S}$-Optimierung, (a) & (b) ursprüngliche Dosis- /Wirkungs-Kurven, (c) & (d) $\hat{S}$-optimierte Kurven, Akt3-Assay, PV- Datensatz,

Figur 16    $\hat{S}$-Wert bei $Z'$-Optimierung des Akt3-Assays, links Whatman- Datensatz, rechts PV-Datensatz,

Figur 17    $Z'$-Wert bei $\hat{S}$-Optimierung des Akt3-Assays, links Whatman- Datensatz, rechts PV-Datensatz,

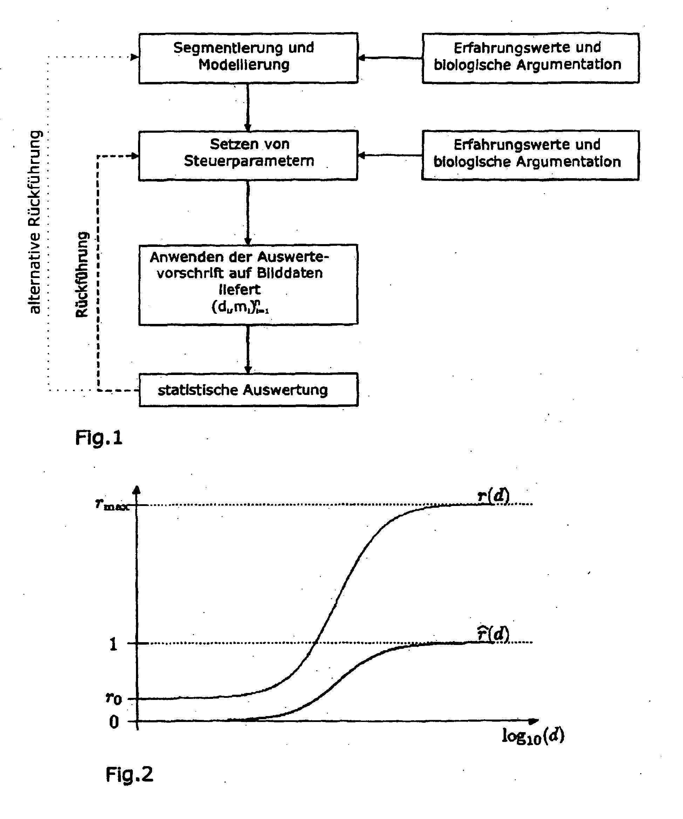

Figur 1.8    $\hat{S}$-Optimierung der U20S-Zellen des $ET_A$R-Assays, Datensatz 070116_Platte1, links nicht optimierte Dosis-/ Wirkungs-Kurve, rechts optimierte Dosis-/Wirkungs-Kurve,

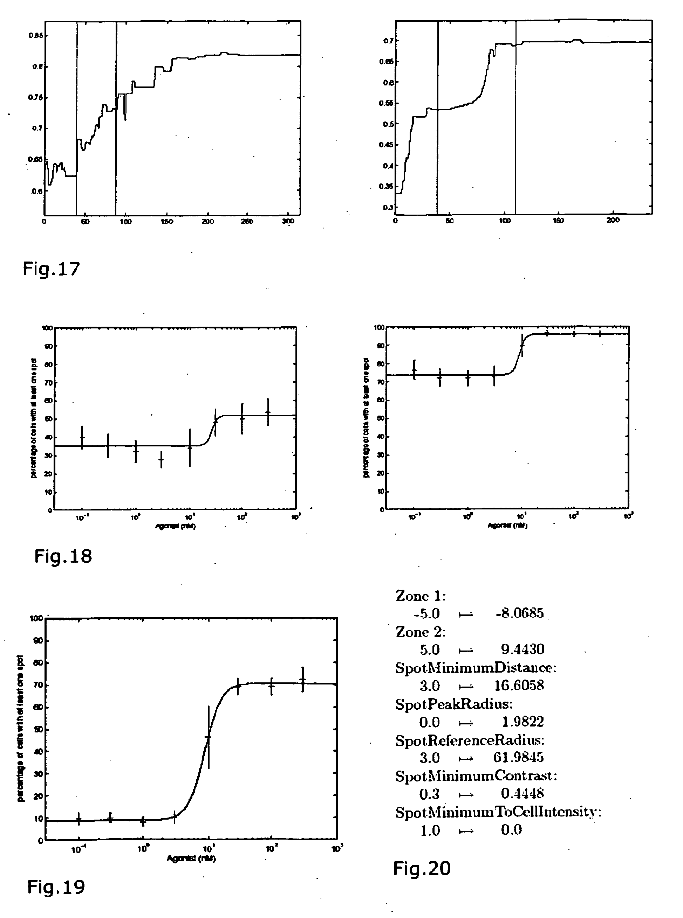

Figur 19    Dosis-/Wirkungs-Kurve nach $\hat{S}$-Optimierung mit Strafterm der U20S-Zellen, Datensatz 070116_Plattel,

Figur 20    $\hat{S}$-optimierte Parameter mit Strafterm der U2OS-Zellen Datensatz 070116_Plattel,

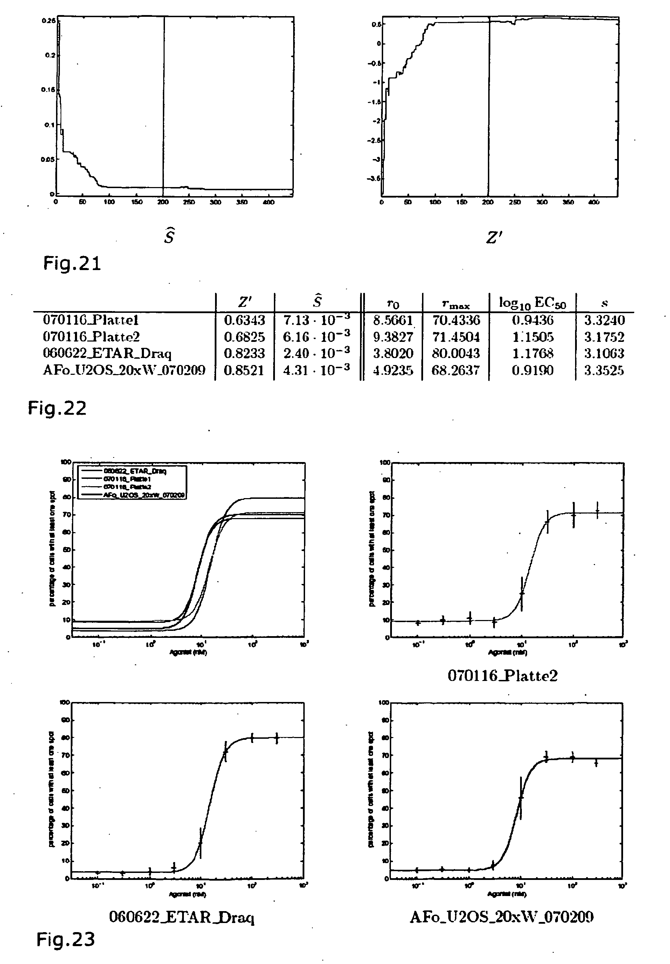

Figur 21    Tabelle der Entwicklung von $\hat{S}$- und $Z'$-Wert während der $\hat{S}$- Optimierung mit Strafterm der U2OS-Zellen Datensatz 070116_Platte1,

Figur 22    Anwendung der bezüglich des Datensatzes 070116_Plattel optimierten Parameter auf die U2OS-Daten des $ET_A$R-Assays,

Figur 23    Anwendung der bezüglich des Datensatzes 070116_Platte1 optimierten Parameter auf die anderen U2OS-Daten des $ET_A$R- Assays,

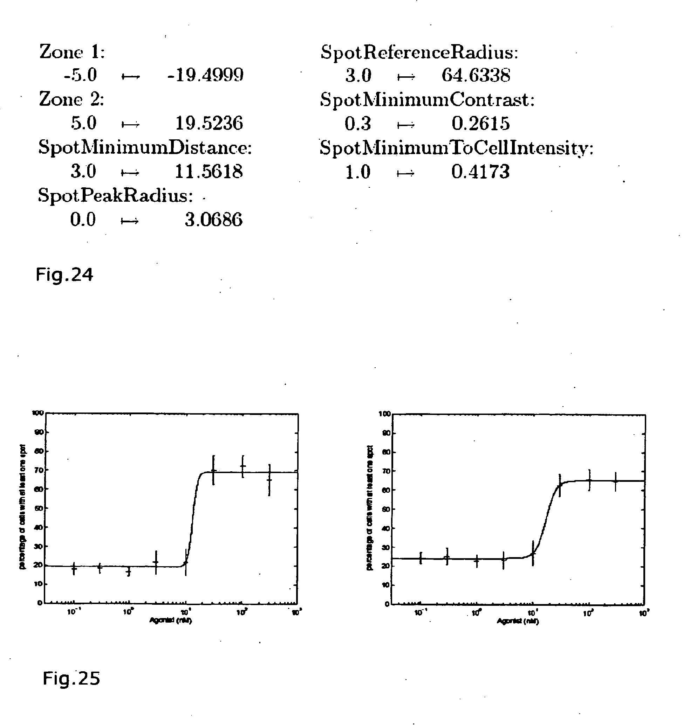

Figur 24    Tabelle der $\hat{S}$-optimierten Parameter mit Strafterm der CHO- Zellen, Datensatz Afo_080129_CHO_ETAR und

Figur 25    Dosis-/Wirkungs-Kurven nach $\hat{S}$-Optimierung mit Strafterm der CHO-Zellen

Der erfindungsgemäße Grundgedanke der Parameteroptimierung für die High-Content-Analyse beim Hochdurchsatz-Screening ist, die einstellbaren Steuerparameter mit Hilfe der Ergebnisse der statistischen Auswertung durch eine Rückführung iterativ zu verbessern, möglicherweise ohne dass die Parameter aufgrund biologischer Begründungen voreingestellt wurden (Figur 1).

Nachfolgend wird erläutert, was unter einer "optimalen Einstellung" zu verstehen ist und es werden geeignete statistische Zielfunktionen definiert.

Ausgangspunkt ist wie vorgehend beschrieben eine Mehrzahl von Proben, die bevorzugt in einzelnen Probenkammern ("Wells") eines Mehrkammerprobenträgers vorliegen. Grundlage der folgenden statistischen Analysen ist jeweils ein Aktivitätswert, nachfolgend auch als "Well-Maß" bezeichnet, der aus einer Messung an einer Probe mit bekannter Testsubstanz-Konzentration mittels der Auswertevorschrift bestimmt wird.

$Z'$-Wert:

Der $Z'$-Wert ist ein statistisches Kennzeichen, das eine Aussage über die Qualität des Assays macht. Er basiert auf Weil-Maßen, die an Referenzproben mit Testsubstanzen bekannt hoher Aktivität ("positive Kontrolle") bzw. ohne Testsubstanz ("negative Kontrolle") ermittelt wurden.

$$Z' := 1 - 3\frac{\sigma_{\mathrm{pos}} + \sigma_{\mathrm{neg}}}{|\mu_{\mathrm{pos}} - \mu_{\mathrm{neg}}|},$$

wobei $\mu_{pos}, \mu_{neg}$ und $O_{pos}, O_{neg}$ die Mittelwerte und Standardabweichungen der Well-Maße der positiven bzw. negativen Kontroll-Wells sind. Für $Z'$ gilt offensichtlich $Z' \leq 1$. Die Qualität des Assays ist um so besser, je näher $Z'$ der 1 kommt und damit ist $Z'$ eine geeignete Zielfunktion. Es ergibt sich das Optimierungsproblem

$$\min_{x \in \mathbb{R}^n} f(x) \quad \text{mit} \quad f(x) := -Z'(x)$$

$$(2.1)$$

mit $n$ stetigen Parametern $x \in R''$. Die Kosten einer Auswertung von $f = -Z'$ sind relativ hoch, da zum Beispiel beim Akt3-Assay 16 Kontroll-Wells, d.h. 96 Bilder, ausgewertet werden müssen.

Dosis-Wirkungs-Kurve:

Ein gängiges Instrument zur Analyse eines Assays mit verschiedenen Dosen ist die Dosis-Wirkungs-Kurve. Sie stellt den Zusammenhang zwischen Substanzaktivität und der Dosis des Wirkstoffs dar. Der vollständige Zusammenhang von Dosis und Wirkung wird häufig erst durch Aufspannen mehrerer Größenordnungen der Dosis darstellbar, daher ist es üblich diese logarithmisch darzustellen.

Je nachdem welcher Wirkstoff eingesetzt wird und welches Maß zur Beschreibung der Wirkung genutzt wird, kann eine Dosis-Wirkungs-Kurve fast jede Form haben, allerdings folgen die meisten Kurven einer Standardform. Diese Standard-Dosis-Wirkungs-Kurve heißt Hill-Equation und ist eine von vier Parametern abhängige logistische Funktion. Sie ist definiert als

$$r(d) := \begin{cases} r_0 + \dfrac{r_{max} - r_0}{1 + \left(\dfrac{EC_{50}}{d}\right)^s} = r_0 + \dfrac{r_{max} - r_0}{1 + 10^{s(\log_{10} EC_{50} - \log_{10}(d))}}, & \text{für } d > 0, \\ r_0, & \text{für } d = 0, \end{cases}$$

$$(\text{Hill-Equation})$$

wobei $r_0$ den Zustand ohne Stimulation und $r_{max}$ die maximale Wirkung bezeichnen. Der Parameter $s$ beeinflusst die Neigung (Hill-Slope) der Kurve und $EC_{50}$ ist die Dosierung, welche die "halbe Wirkung" $\dfrac{r_0 + r_{max}}{2}$ hervorruft. Wir werden uns auf dieses Dosis-Wirkungs-Modell beschränken. Die vier Parameter $r_0$, $r_{max}$, $s$ und $EC_{50}$ können mit der Methode der kleinsten Quadrate mit Hilfe nichtlinearer Regression bestimmt werden:

Seien $m_1, ..., m_n$ die gemessenen Well-Maße mit zugehörigen Dosen $d_1, ..., d_n$. Um die Reproduzierbarkeit zu gewährleisten, werden häufig die gleichen Dosen mehrmals getestet, so dass die $d_i$ nicht zwingend paarweise verschieden sein müssen. Zur Bestimmung der Ausgleichskurve $r(d)$ lösen wir dann das Minimierungsproblem

$$S := \frac{1}{n} \sum_{i=1}^{n} (m_i - r(d_i))^2 \rightarrow \min_{r_0, r_{max}, s, EC_{50}} .$$

Das Residuum $S$ gibt die mittlere quadratische Abweichung zur Modellkurve an.

Zur Lösung dieser nichtlinearen Regression wird vorstehende Gleichung iterativ mit geeigneten Startwerten für $r_0$, $r_{max}$, $s$ und $EC_{50}$ gelöst. Die Wahl dieser Werte kann die Konvergenz des. Lösungsverfahrens beeinflussen und im schlimmsten Fall kann aus einer schlechten Wahl sogar die Divergenz des Verfahrens resultieren.

Für die Hill-Equation ist es möglich, durch Interpretation der Parameter $r_0$, $r_{max}$, $s$ und $EC_{50}$ an geeignete Startparameter zu gelangen. Als Startwerte für die minimale und maximale Wirkung $r_0$ und $r_{max}$ wird gewählt:

$$r_0 = \min_{i=1,...,n} (m_i) - \varepsilon \quad \text{bzw.} \quad r_{max} = \max_{i=1,...,n} (m_i) + \varepsilon$$

mit einem kleinen $\varepsilon > 0$. Damit folgt für $d > 0$, $r_0 < r < r_{max}$

$$r = r_0 + \frac{r_{max}-r_0}{1+10^{s(\log_{10} EC_{50} - \log_{10}(d))}} = \frac{r_0 \cdot 10^{s(\log_{10} EC_{50} - \log_{10}(d))}+r_{max}}{1+10^{s(\log_{10} EC_{50} - \log_{10}(d))}}$$

$$\Leftrightarrow \quad r - r_{max} = (r_0 - r)10^{s(\log_{10} EC_{50} - \log_{10}(d))}$$

$$\Leftrightarrow \quad \underbrace{\log_{10}\left(\frac{r - r_{max}}{r_0 - r}\right)}_{=:\tilde{r}} = s(\log_{10} EC_{50} - \log_{10}(d))$$

und daher verhält sich $\tilde{r}$ linear bzgl. $\log_{10}(d)$. Wegen der Definitionen gilt $\dfrac{m_i - r_{max}}{r_0 - m_i} > 0,$ , $i=1, ..., n$, und somit ist

$$\tilde{m}_i := \log_{10}\left(\frac{m_i - r_{max}}{r_0 - m_i}\right) \quad \text{für } i=1, ..., n \text{ definiert.}$$

Die lineare Regression

$$\sum_{i=1}^{n}(\tilde{m}_i - \tilde{r}(d_i))^2 = \sum_{i=1}^{n}\left(\log_{10}\left(\frac{m_i - r_{max}}{r_0 - m_i}\right) - s(\log_{10} EC_{50} - \log_{10}(d))\right)^2 \to \min_{s, EC_{50}}$$

liefert Startwerte

$$s = -\frac{\sum_{i=1}^{n}(\log_{10}(d_i) - \mu_{\log_{10}(d)})(\tilde{m}_i - \mu_{\tilde{m}})}{\sum_{i=1}^{n}(\log_{10}(d_i) - \mu_{\log_{10}(d)})^2},$$

$$\log_{10} EC_{50} = \frac{\mu_{\tilde{m}} + s\mu_{\log_{10}(d)}}{s},$$

mit den Mittelwerten $\mu_{\log_{10}(d)} := \dfrac{1}{n}\sum_{i=1}^{n}\log_{10}(d_i)$ und $\mu_{\tilde{m}} := \dfrac{1}{n}\sum_{i=1}^{n}\tilde{m}_i$ .

$\hat{S}$-wert:

Erfindungsgemäß, kann auch das Residuum der Dosis-/Wirkungs-Regression zur Optimierung genutzt werden. Für $n$ gemessene Well-Maße $m_i(i=1...n)$ mit zugehörigen Dosen $d_i(i=1...n)$ kurz $(d_i,\ m_i)_{i=1}^{n}$ ergibt sich durch nicht-lineare Regression die Dosis-/Wirkungs-Kurve

$$r : \mathbb{R} \longrightarrow \mathbb{R}, \qquad d \longmapsto r_0 + \frac{r_{\max} - r_0}{1 + \left(\frac{EC_{50}}{d}\right)^s}.$$

Ein kleines Residuum

$$S := \frac{1}{n}\sum_{i=1}^{n}(m_i - r(d_i))^2$$

ist ein Hinweis auf eine gute Anpassung an das Hill-Equation-Modell. Das Residuum $\hat{S}$ hängt allerdings sehr entscheidend von der Spannweite der Kurve $|r_{\max} - r_0|$ ab. Daher wird vorzugsweise nach der Regression eine Normierung der Dosis-/Wirkungs-Kurve und der Well-Maße durchgeführt

$$\hat{r}(d) := \frac{r(d) - r_0}{|r_{\max} - r_0|}; \quad \hat{m}_i := \frac{m_i - r_0}{|r_{\max} - r_0|}, i = 1, \ldots, n.$$

Die Größe $\dfrac{\hat{r}(d)}{100}$ gibt uns jetzt an, zu wie viel % eine durchschnittliche Zelle bei der Dosis d angeregt ist. Das zu $\hat{r}$ gehörende Residuum $\hat{S}$ verhält sich wie folgt:

$$\hat{S} := \frac{1}{n}\sum_{i=1}^{n}(\hat{m}_i - \hat{r}(d_i))^2 = \frac{1}{n}\sum_{i=1}^{n}\left(\frac{m_i - r_0}{|r_{\max} - r_0|} - \frac{r(d_i) - r_0}{|r_{\max} - r_0|}\right)^2$$

$$= \frac{1}{(r_{\max} - r_0)^2}\frac{1}{n}\sum_{i=1}^{n}(m_i - r(d_i))^2 = \frac{1}{(r_{\max} - r_0)^2}S.$$

Das nun hergeleitete Residuum der normierten Dosis-/Wirkungs-Kurve

$$\widehat{S} = \frac{1}{(r_{\max} - r_0)^2}\, S$$

eignet sich für die Optimierung ebenfalls, da es durch die Normierung (siehe Figur 2) unabhängig von der Spannweite $|r_{max} - r_0|$ ist und ein kleines Residuum eine gute Anpassung an das Hill-Equation-Modell bedeutet. Die Größe $|r_{max} - r_0|$ wird in diesem Zusammenhang auch *dynamic range* genannt. Ein weiteres sinnvolles Optimierungsproblem lautet also

$$\min_{x \in \mathbb{R}^n} f(x) \quad \text{mit} \quad f(x) := \widehat{S}(x),$$

$$(2.2)$$

mit $n$ stetigen Parametern $x \in R^n$, Man beachte, dass diese Optimierung sehr viel aufwändiger ist als Gleichung (2.1), da für eine Funktionsauswertung von $f = \widehat{S}$ nicht nur die Kontroll-Wells, sondern alle Wells analysiert werden müssen.

Zusammenhang von $Z'$- und $\widehat{S}$-Wert:

Bei der Berechnung des $Z'$-Wertes werden nur die Kontroll-Wells, d.h. Signale mit minimaler und maximaler Amplitude verwendet. Im Gegensatz dazu fließen in den $\widehat{S}$-Wert neben Signalen ohne Hinzugabe eines Wirkstoffs und mit maximaler Dosis auch Bilder von Zellen, die nur teilweise angeregt sind, ein. Daher kann es bei der Optimierung bezüglich des $Z'$-Wertes gemäß Gleichung (2.1) passieren, dass zwar die Standardabweichungen in den Kontroll-Wells sehr klein werden, es aber bei mittleren Dosen starke Abweichungen gibt, d.h. das Residuum der Dosis-/Wirkungs-Regression vergrößert wird. Oder anders ausgedruckt: Es kann passieren, dass sich der $\widehat{S}$-Wert während der $Z'$-Wert-Optimierung erheblich verschlechtert.

Minimiert man dagegen $\widehat{S}$ gemäß Gleichung (2.2), so fließen sowohl Signale minimaler und maximaler Wirkung als auch feinere Abstufungen dazwischen in die Optimierung ein. So ist hier eine bedeutende Verschlechterung des $Z'$-Wertes während der $\widehat{S}$-Optimierung nicht zu erwarten.

Als nächstes muss überlegt werden, welche Parameter zur Optimierung zweckmäßig sind. Beim automatischen Einstellen von Segmentierungsparametern darf die Optimierung des Zielfunktionswertes nicht auf Kosten einer guten Objekterkennung geschehen. Eine tadellose Detektion der Objekte ist die Grundvoraussetzung der Zellanalyse.

Weiter erfolgt vorzugsweise keine Optimierung über Exklusionsparameter. Diese Parameter sollen Zellen von der Analyse ausschließen, die nicht optimal aufgenommen oder nicht mehr lebendig sind. Die Aussonderung dieser pathologischen Falle geschieht z.B. über Grenzwerte der Größe und Intensitäten. Eine Optimierung über diese Parameter ist ebenfalls nicht erwünscht, da pathologische Zellen die Analyse nicht beeinflussen sollen. Die Exklusionsparameter werden vorzugsweise aufgrund biologischer Überlegungen festgelegt.

Damit bleiben zur Optimierung noch alle Parameter, die in das die Aktivität der Zelle beschreibende Modell einfließen.

Bei der Wahl der Optimierungsroutine muss erfindungsgemäß vorzugsweise Folgendes beachtet werden:

i) Die zu minimierenden Zielfunktionen $-Z'$ bzw. $-\widehat{S}$ sind eventuell nicht bzgl. x differenzierbar.

ii) Eine Funktionsauswertung ist im Allgemeinen sehr teuer. Für eine Auswertung von $Z'$ müssen die Kontroll-Wells und für die Auswertung von $\widehat{S}$ sogar alle Wells analysiert werden.

Die erste Eigenschaft von $f$ impliziert, dass ein ableitungsfreies Verfahren genutzt werden muss. Auch eine Approximation des Gradienten mit Hilfe finiter Differenzen wäre möglicherweise nicht zufriedenstellend, falls $f$ viele lokale Minima hat. Die zweite Einschränkung bei der Wahl eines Optimierungsverfahrens lässt sich etwas abschwächen, da die Bilder unabhängig voneinander analysiert werden können und damit eine Aufteilung der Daten (in Wells oder sogar Subwells) möglich ist. So könnte $f$ leicht auf mehreren Rechnern parallel ausgewertet werden. Eine weitere Abschwächung ist, dass die Segmentierung nur einmal durchgeführt werden braucht, da nicht über Segmentierungsparameter optimiert werden soll. Die Segmentierungsergebnisse können abgespeichert und immer wieder genutzt werden.

Auf Grund des Punktes i) werden zur Optimierung das Verfahren von Nelder und Mead verwenden. Diese robuste ableitungsfreie Methode zur Optimierung nichtlinearer Funktionen eignet sich ausgezeichnet für unsere Optimierungsprobleme. Es sei hier bemerkt, dass es in dieser Arbeit nicht darum geht, die bestmögliche Optimierungsroutine zu finden. Vielmehr soll statt dessen eine sinnvolle Zielfunktion zur automatischen Verbesserung der Qualität von High-Throughput-Screenings hergeleitet werden und die Praktikabilität der Parameteroptimierung gemäß Figur 1 an Beispielassays demonstriert werden.

Das Verfahren von Nelder-Mead ist ein heuristisch motiviertes, ableitungsfreies Minimierungsver-fahren. Es nutzt als Grundstruktur ein Simplex aus Annäherungen einer optimalen Lösung und geht iterativ in ein Simplex mit besseren Zielfunktionswerten über. Die Darstellung richtet sich nach Alt, W.: Nichtlineare Optimierung. Vieweg 2002 und Kelley, C.T.: Iterative Methods for Optimization. Society for Industrial & Applied Mathematics, 1999.

Im Folgenden sollen nun die Optimierungsaufgaben gemäß Gleichung (2.1) und (2.2) auf die Beispielassays angewendet werden. Die Berechnungen wurden auf einem Notebook mit Pentium M Prozessor mit 1.8 GHz durchgeführt und die angegebenen Rechenzeiten beziehen sich immer auf dieses System.

**Beispiel I:**

Akt3-Assay.

Das erste Beispiel der Parameteroptimierung ist der Akt3-Assay, bei dem zunächst der Whatman-Datensatz bearbeitet wird, an Hand dessen Daten die HCS-Analyse entwickelt wurde. Später wurden die Parameter mit Hilfe der Optimierungsprobleme gemäß Gleichung (2.1) und (2.2) so eingestellt, dass das Modell auch für den PV-Datensatz gute Resultate liefert.

Whatman-Datensatz:

Das Modell wurde für den Whatman-Datensatz entwickelt und liefert dort sehr gute Ergebnisse. Es wurde sodann untersucht, ob es möglich ist, die an exemplarischen Zellen definierten Parameter noch besser einzustellen.

Z'-Maximierung:

Der $Z'$-Wert des Whatman-Datensatzes des Akt3-Assays ergab sich als $Z' = 0.6347$. Das zur Beschreibung der Aktivität benutze Maß

$$\text{Zell-Maß} := Q_{q1} \ (\text{Zytoplasmamaske}) - Q_{q2} \ (\text{Membranmaske})$$

hängt von sechs Parametern ab. Dies sind die zwei Größen der Quantile ($q_1 = 0.8$ und $q_2 = 0.9$), die zwei Grenzen der Membranmaske (0 und 4) und die zwei Grenzen der Zytoplasmamaske (7 und 13).

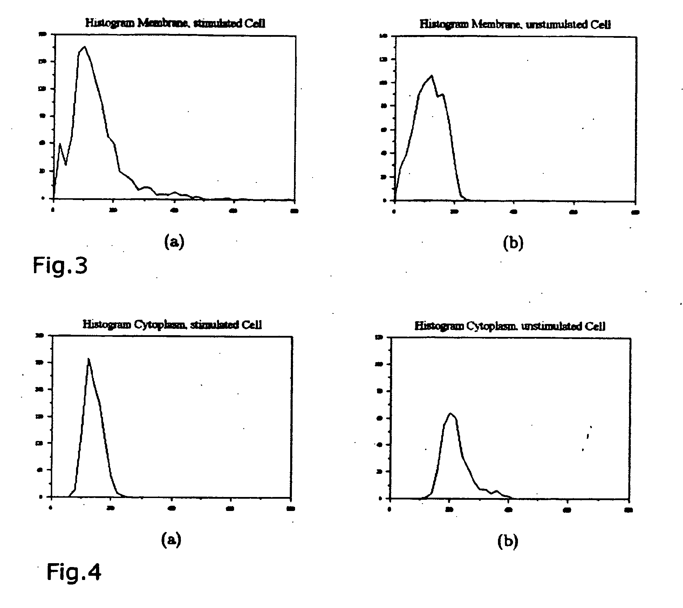

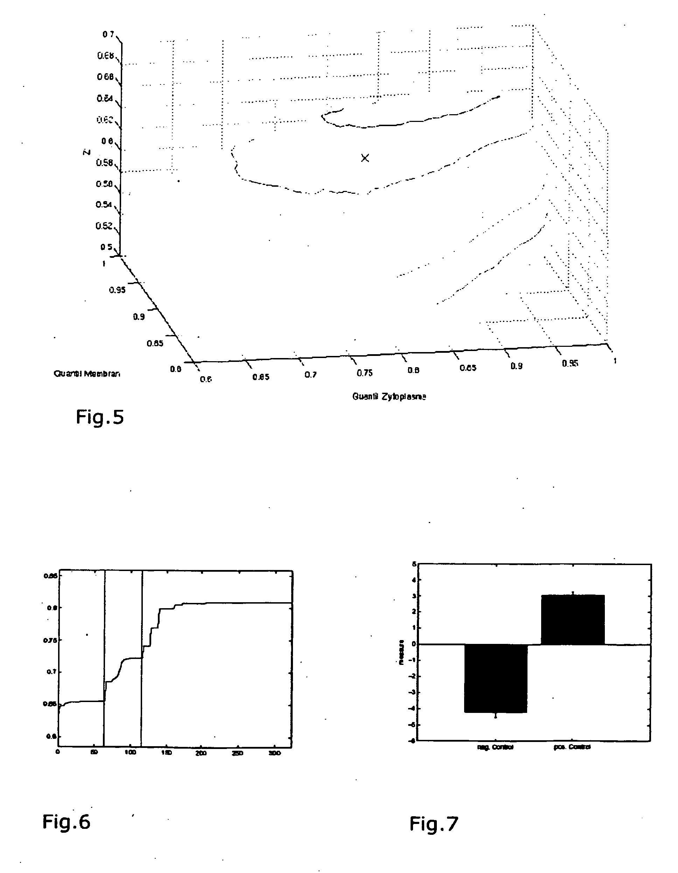

Als erstes wird eine Optimierung der zwei Quantile durchgeführt. Der Algorithmus startet bei $(q_1, q_2)^T = (0.8, 0.9)^T$ und liefert nach 64 Funktionsauswertungen einen Wert von

$$Z' = 0.6552$$

mit den Quantilen $(q_1, q_2)^T = (0.8463, 0.9654)^T$. Da nicht über die Segmentierungsparameter optimiert wird, wurde die Segmentierung nur einmal ausgeführt und die Ergebnisse abgespeichert. Dies reduziert die Kosten einer Funktionsauswertung von $f = Z'$ von fünf Minuten auf ungefähr 30 Sekunden. Es vergingen also während der Optimierung der zwei Quantile ca. 32 Minuten. Scheinbar waren die an Hand der exemplarischen Histogramme (Figur 3 und 4) geschätzten Quantile nicht optimal. Die Figur 5 zeigt den Zusammenhang zwischen den Quantilen und dem $Z'$-Wert, das Kreuz markiert die initialen Parameter $(q_1, q_2)^T = (0.8, 0.9)^T$. Bei der Optimierung der Quantile ist zu beachten, dass das zulässige Gebiet der Parameter $[0, 1]^2 \subseteq R^2$ ist. Das Problem wird durch Projektion auf $[0, 1]^2$ gelöst, d.h. es wird für $x \notin [0, 1]^2$ definiert (siehe Figur 5):

$$Z'(x) := Z'(y^*), \quad \text{mit } \|x - y^*\| = \inf_{y \in [0,1]^2} \|x - y\|.$$

Als nächstes könnten analog alle sechs Parameter optimiert werden. Stattdessen wird das Modell zunächst ein wenig modifiziert, um weitere Parameter zu erhalten. Es wird zur Beschreibung der Aktivität einer Zelle das

$$\text{Zell-Maß} := \lambda \cdot \left( Q_{q_1}(\text{Zytoplasmamaske}) \right)^{\mu_1} - \left( Q_{q_2}(\text{Membranmaske}) \right)^{\mu_2}$$

definiert, mit einem Faktor $\lambda$ und den Potenzen $\mu_1$ und $\mu_2$. Das Modell hangt von neun Variablen ab und ist eine Verallgemeinerung von Gleichung (1.1). Zunächst wird der Faktor $\lambda = 1$ und die Potenzen $\mu_1$ und $\mu_2 = 1$ gesetzt.

Der nächste Schritt soll die Optimierung dieses neuen Modells bezüglich der neun Parameter sein. Es hat sich bei den Zielfunktionen (2.1) und (2.2) als vorteilhaft erwiesen, die Parameter Schritt für Schritt zu optimieren, da das Ergebnis des Nelder-Mead-Algorithmus empfindlich von den Startparametern abhängt. Daher werden als nächstes ausschließlich die Potenzen $\mu 1$ und $\mu_2$ optimiert und die restlichen Parameter werden festgehalten. Nach 51 Funktionsauswertungen und knapp 26 Minuten ergab die Optimierung

$$Z' = 0.7227,$$

mit Potenzen $(\mu_1, \mu_2)^T = (0.5223, 0.5093)^T$.

Der letzte Schritt ist die Optimierung aller neun Parameter. Als Startwerte wählen wir die schon voreingestellten Quantile $(q_1, q_2)^T = (0.8463, 0.9654)^T$ und Potenzen $(\mu_1, \mu_2)^T = (0.5223, 0.5093)^T$.

Die Grenzen der Membran- und Zytoplasmamaske starten bei $(0, 4)^T$ bzw. $(7,13)^T$, der Faktor $\lambda$ bei 1. Nach weiteren 207 Funktionsauswertungen ergibt sich so

$$Z' = 0.8096.$$

Die Steuerparameter haben sich durch die Optimierung wie folgt geändert:

| | | | | | | |
|---|---|---|---|---|---|---|
| $q_1 = 0.8$ | $\rightarrow$ | 0.5911 | Zone 1: | 0 | $\rightarrow$ | 2.0723 |
| $q_2 = 0.9$ | $\rightarrow$ | 1.0 | | 4 | $\rightarrow$ | 4.6498 |
| $\mu_1 = 1.0$ | $\rightarrow$ | 0.5183 | Zone 2: | 7 | $\rightarrow$ | 7.161 |
| $\mu_2 = 1.0$ | $\rightarrow$ | 0.5108 | | 13 | $\rightarrow$ | 11.5971 |
| $\lambda = 1.0$ | $\rightarrow$ | 1.3341 | | | | |

Insgesamt dauerte die dreistufige Optimierung mit 322 Funktionsauswertungen ca. 2.5 Stunden und der *Z'-Wert* verbesserte sich dabei sehr deutlich. Die Figur 6 stellt noch einmal die Auswertung der Kontroll-Wells des optimierten Maßes dar, in Figur 7 ist die Entwicklung des *Z'*-Wertes in Abhängigkeit der Funktionsauswertungen dargestellt. Die roten Markierungen stellen die drei Stufen dar.

$\hat{S}$-Minimierung:

Nachdem der *Z'*-Wert deutlich verbessert wurde, wird nun für den $\hat{S}$-Wert analog vorgegangen. Die nachstehende Tabelle zeigt, in welchen Dosen die verwendeten Substanzen eingesetzt wurden.

|   | 1 | 2 | 3 | 4 | 5 | 6 | 7 | 8 | 9 | 10 | 11 | 12 |
|---|---|---|---|---|---|---|---|---|---|---|---|---|
| **A** | Neg.Ctrl. | 0.0 | 2.0 | 3.9 | 7.8 | 15.6 | 31.3 | 62.5 | 125 | 250 | 500 | Pos.Ctrl. |
| **B** | Neg.Ctrl. | 0.0 | 2.0 | 3.9 | 7.8 | 15.6 | 31.3 | 62.5 | 125 | 250 | 500 | Pos.Ctrl. |
| **C** | Neg.Ctrl. | 0.0 | 2.0 | 3.9 | 7.8 | 15.6 | 31.3 | 62.5 | 125 | 250 | 500 | Pos.Ctrl. |
| **D** | Neg.Ctrl. | 0.0 | 2.0 | 3.9 | 7.8 | 15-6 | 31-3 | 62.5 | 125 | 250 | 500 | Pos-Ctrl |
| **E** | Neg.Ctrl. | 0.0 | 0.3 | 0.6 | 1.3 | 2-5 | 5 | 10 | 20 | 40 | 80 | Neg.Ctrl. |
| **F** | Neg.Ctrl. | 0.0 | 0.3 | 0.6 | 1.3 | 2.5 | 5 | 10 | 20 | 40 | 80 | Neg.Ctrl. |
| **G** | Neg.Ctrl. | 0.0 | 0.3 | 0.6 | 1.3 | 2.5 | 5 | 10 | 20 | 40 | 80 | Neg.Ctrl. |
| **H** | Neg.Ctrl. | 0.0 | 0.3 | 0.6 | 1.3 | 2.5 | 5 | 10 | 20 | 40 | 80 | Neg Ctrl. |
| Neg. Control = 0.25% DMSO,<br>Pos. Control = 300 *nM* Wortmannin | | | | | | | | | | | | |

Wie vorstehend angemerkt, wird für die Dosis-/Wirkungs-Analyse nur die Spalten 2 bis 11 der Mikrotiterplatte genutzt. Die Spalten 1 und 12 werden nicht verwendet, da bei der $\hat{S}$-Wert-Minimierung alle Dosen gleich gewichtet werden sollen und für die positiven Kontroll-Signale nur der Antagonist Wortmannin verwendet wurde. So fallen pro Dosis und Wirkstoff 4 Datenpunkte an und zur Berechnung der Dosis-/Wirkungs-Kurven müssen je Wirkstoff 40 Well-Maße untersucht werden.

Die Optimierung ist wieder dreistufig geschehen, es werden hier allerdings nur die Ergebnisse nach dem dritten Schritt dargestellt. Die Kosten einer Auswertung von $f = \hat{S}$ betragen ca. vier Minuten, wobei die Segmentierung wieder einmalig ausgeführt und die Segmentierungsergebnisse zur Wiederverwendung abgespeichert wurden. Bei dieser Optimierung, die sehr viel aufwändiger ist als die *Z'*-Maximierung, ist erst durch dieses Vorgehen eine Durchführung in einer akzeptablen Zeit möglich.

Der $\hat{S}$-Wert des Whatman-Datensatzes des Akt3-Assays ergibt sich vor der Optimierung als

$$\widehat{S} = 1.01 \cdot 10^{-2}$$

wobei er sich als Summe der Einzelresiduen $\hat{S}_{\text{wortmanntn}}$ und $\hat{S}_{\text{LY294002}}$ der Substanzen Wortmannin und LY294002 zusammensetzt. Die Einzelresiduen ergeben sich als

$$\widehat{S}_{\text{Wortmannin}} = 7.28 \cdot 10^{-3}, \quad \widehat{S}_{\text{LY294002}} = 2.79 \cdot 10^{-3}$$

Die Minimierung von $\hat{S}$ ergab nach 314 Funktionsauswertungen und ca. 21 Stunden

$$\widehat{S} = 4.15 \cdot 10^{-3}$$

mit

$$\widehat{S}_{\text{Wortmannin}} = 3.29 \cdot 10^{-3}, \quad \widehat{S}_{\text{LY294002}} = 8.58 \cdot 10^{-4}.$$

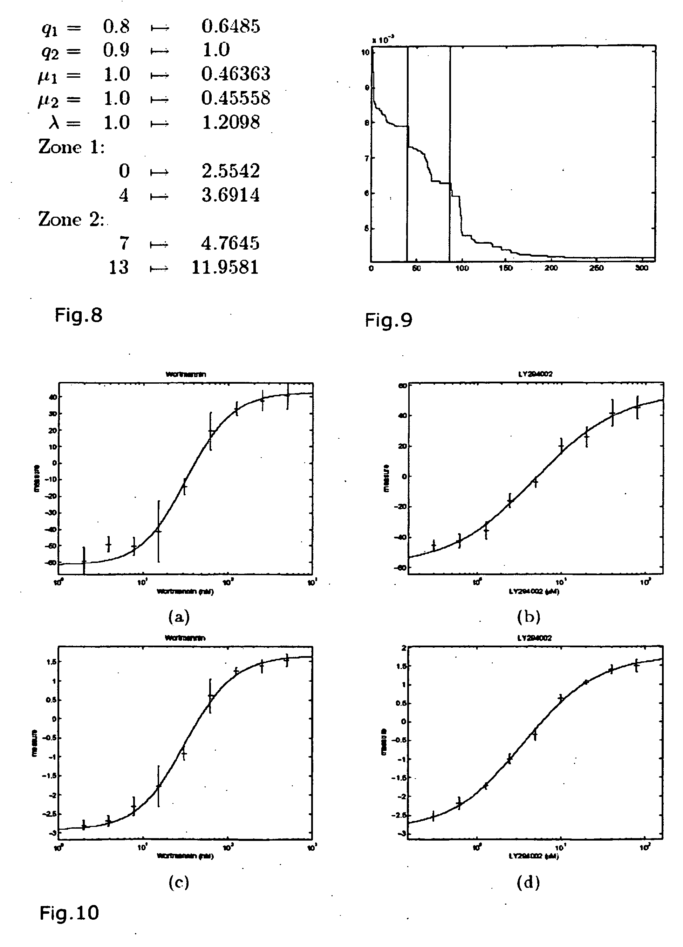

Die Veränderung der Parameter durch die $\widehat{S}$-Optimierung ist in der in Figur 8 gezeigten Tabelle dargestellt. Die Figur 9 zeigt die zeitliche Entwicklung von $\widehat{S}$ während der Optimierung und in Figur 10 ist das Endergebnis grafisch als Dosis-/Wirkungs-Kurve dargestellt. In der ersten Zeile sind noch einmal die ursprünglichen Dosis-/Wirkungs-Kurven und in der zweiten Zeile die optimierten Kurven zu sehen. Die Weit-Maße sind nach der Optimierung deutlich besser an das Hill-Equation-Modell angepasst.

PV-Datensatz:

Im Gegensatz zum Whatman-Datensatz ist die Qualität des PV-Datensatzes mit $Z' = 0.3314$ nicht zufriedenstellend. Da das Modell gemäß Gleichung (1.1) zur Analyse des Akt3-Assay für den Whatman-Datensatz hergeleitet wurde und sich die Datensatze in Kontrast und Helligkeit stark unterscheiden, ist der schlechte $Z'$-Wert allerdings auch nicht verwunderlich. Als nächstes wurde versucht, die Parametereinstellungen automatisch so anzupassen, dass das Modell ebenfalls zur Analyse des PV-Datensatzes geeignet ist. Die für den Whatman-Datensatz verwendete Parameteroptimierung wurde analog auf den PV-Datensatz angewendet und die Ergebnisse in Figur 10 dargestellt.

$Z'$-Maximierung:

Die dreistufige Optimierung des $Z'$-Wertes ergab nach 674 Funktionsauswertungen und etwas mehr als 5.5 Stunden

$$Z' = 0.7878.$$

Die Parameter haben sich durch die Optimierung wie folgt geändert:

| | | | | | | | |
|---|---|---|---|---|---|---|---|
| $q_1 =$ | 0.8 | $\rightarrow$ | 0.0 | Zone 1: | 0 | $\rightarrow$ | -0.9840 |
| $q_2 =$ | 0.9 | $\rightarrow$ | 1.0 | | 4 | $\rightarrow$ | 1.4675 |
| $\mu_1 =$ | 1.0 | $\rightarrow$ | 0.2129 | Zone 2: | 7 | $\rightarrow$ | 7.0288 |
| $\mu_2 =$ | 1.0 | $\rightarrow$ | 0.1398 | | 13 | $\rightarrow$ | 14.3346 |
| $\lambda =$ | 1.0 | $\rightarrow$ | 1.2203 | | | | |

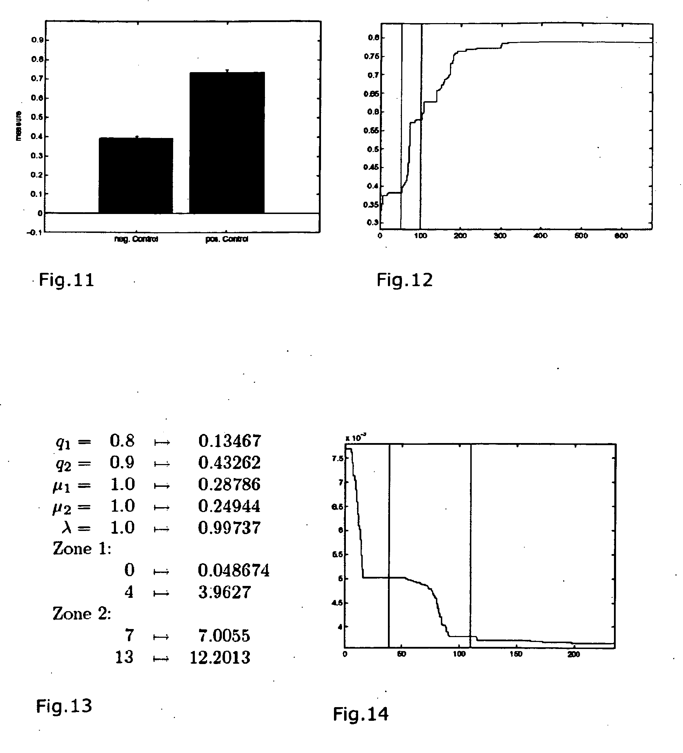

Die Figur 11 stellt die Auswertung der Kontroll-Wells des optimierten Modells dar, in Figur 12 ist die Entwicklung des $Z'$-Wertes in Abhängigkeit der Funktionsauswertungen dargestellt. Die Markierungen stellen wieder die drei Stufen dar.

$\widehat{S}$-Minimierung:

Der $\widehat{S}$-Wert des PV-Datensatzes des Akt3-Assays ergibt sich vor der Optimierung als

$$\widehat{S} = 7.70 \cdot 10^{-3}$$

wobei er sich als Summe der Einzelresiduen

$$\widehat{S}_{\text{Wortmannin}} = 4.60 \cdot 10^{-3} \quad \text{und} \quad \widehat{S}_{\text{LY294002}} = 3.10 \cdot 10^{-3}$$

der Substanzen Wortmain und LY294002 zusammensetzt. Die Minimierung von $\overset{\wedge}{S}$ ergab nach 235 Funktionsauswertungen und ca. 15.5 Stunden

$$\widehat{S} = 3.67 \cdot 10^{-3}$$

mit

$$\widehat{S}_{\text{Wortmannin}} = 2.34 \cdot 10^{-3}, \quad \widehat{S}_{\text{LY294002}} = 1.33 \cdot 10^{-3}$$

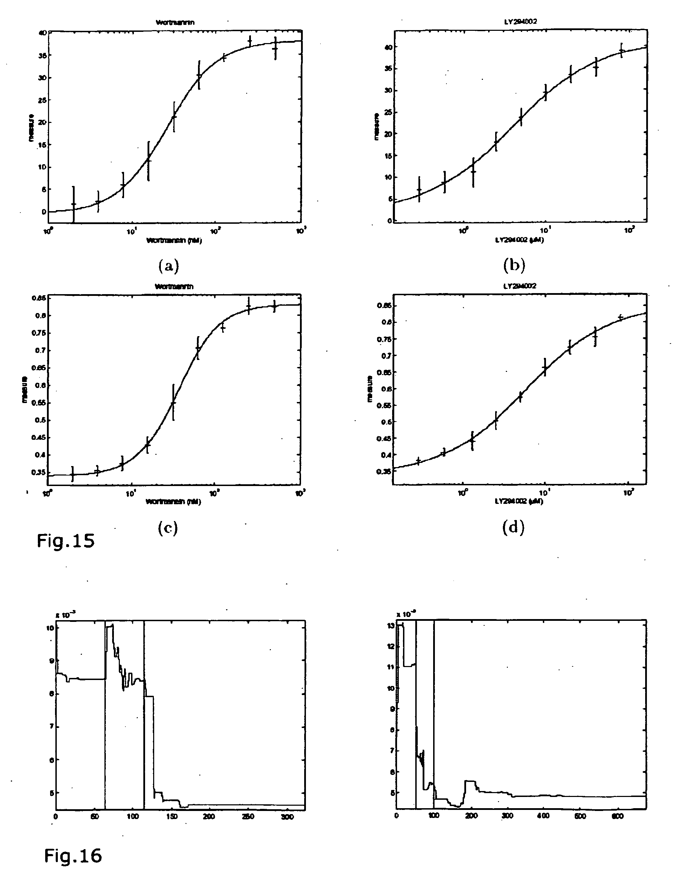

Die Veränderung der Parameter durch die $\overset{\wedge}{S}$-Optimierung ist in der in Figur 13 gezeigten Tabelle dargestellt. Die Figur 14 zeigt die zeitliche Entwicklung von $\overset{\wedge}{S}$ während der Optimierung und in Figur 15 ist das Endergebnis grafisch als Dosis-/Wirkungs-Kurve dargestellt.

Figur 16 stellt den zeitlichen Verlauf des $\overset{\wedge}{S}$-Wertes bei der $Z'$-Optimierung dar. Während der $Z'$-Wert wie erwartet monoton steigt (vgl. Figur 6 bzw. Figur 12), verändert sich der $\overset{\wedge}{S}$-Wert teilweise sehr stark nachteilig. Analog ist in Figur 17 der $Z'$-Wert bei der $\overset{\wedge}{S}$-Minimierung dargestellt. Auch hier sind teilweise Entwicklungen der nicht optimierten Größe zum Schlechteren zu erkennen, allerdings bei Weitem nicht so stark wie bei der $Z'$-Optimierung.

**Beispiel II:**

$ET_AR$-Assay:

Die Acapella-Default-Parameter des Spot-Detektions-Moduls lieferten für die Anwendung auf den $ET_AR$-Assay schlechte Ergebnisse. Anstatt diese von Hand auf Grund von Erfahrungswerten und biologischer Argumentation einzustellen, werden die Parameteroptimierung mit Hilfe des $\overset{\wedge}{S}$-Wertes nach Gleichung (2.2) genutzt. Die Parameteroptimierung des $ET_AR$-Assays ist damit eine sehr viel größere Herausforderung als die des Akt3-Assays, da zum einen mit einem sehr schlechten Startwert begonnen wird und da zum anderen keine Informationen über die Funktionsweise des Moduls benutzt werden soll.

U2OS-Zellen:

Die $\overset{\wedge}{S}$-Minimierung wird zunächst auf den Datensatz 070116_Plattel der U2OS-Zell angewandt. Das Modell hängt von sieben Parametern ab: Fünf Parameter beeinflussen das Spot-Detektions-Modul und zwei weitere Parameter erhält man durch die Definition des Suchbereichs für Spots. In Kapitel 1.3.2 ist diese als (-5, 5) definiert. Die Optimierung soll wieder mehrstufig passieren. Zunächst werden die fünf Parameter des Spot-Detektions-Moduls voreingestellt. In einem zweiten Schritt fließen dann alle Parameter in die Optimierung ein.

Der $\overset{\wedge}{S}$-Wert ergibt sich vor der Optimierung als

$$\hat{S} = 0.2470.$$

Die Minimierung ergab nach 264 Funktionsauswertungen und ca. 15.5 Stunden (d.h. ca. 3.5 Minuten pro Auswertung)

$$\hat{S} = 0.0480.$$

In Figur 18 sind die nicht-optimierte Dosis-/Wirkungs-Kurve links und die optimierte Kurve rechts dargestellt.

Der $\hat{S}$-Wert hat sich zwar deutlich verbessert und das ist auch an den Fehlerbalken in Figur 18 zu erkennen, aber das Ergebnis ist nicht so, wie man es erwartet. Nach der Beschreibung des Assays müssten für geringe Dosen fast keine Zellen einen Spot besitzen und für sehr hohe Dosen fast jede Zelle mindestens einen Spot. Das merkwürdige Ergebnis der Optimierung liegt vielleicht daran, dass das Residuum auch dann klein ist, wenn im Spot-Detektions-Modul solch extreme Schwellwerte eingestellt werden, dass alle Maße nach 0% oder 100% gedruckt werden. Falls die Maße nahe dieser Grenzen liegen, kann es offensichtlich nicht mehr so große Abweichungen voneinander geben. Scheinbar ist man in einem lokalen Minimum gelandet, das stark von der gesuchten Parameter-Einstellung abweicht.

Um dieses Phänomen zu verhindern, wurde das Vorwissen genutzt, dass unstimulierte Zellen keine intrazelluläre Fluoreszenz besitzen wohl aber fast alle stimulierten Zellen. Diese Kenntnis wird in Form eines Strafterms in die Optimierung integriert und der für den $ET_AR$-Assay angepassten Wert

$$\widehat{S}_{ET_AR} := \widehat{S} - \lambda \left( r_{max} - r_0 \right)^2$$

$$(2.3)$$

definiert, mit einer Konstante $\lambda > 0$. Die neue Zielfunktion belohnt große Spannweiten $|r_{max} - r_0|$ bzw. bestraft kleine. Es wird $\lambda > 0$ nunmehr so gewählt, dass

$$\widehat{S} \approx \lambda \left( r_{max} - r_0 \right)^2.$$

Die Minimierung der Zielfunktion (2.3) mit $\lambda = 0.001$ ergab nach 445 Funktionsauswertungen und 26 Stunden

$$\widehat{S} = 7.13 \cdot 10^{-3} \quad \text{und} \quad Z' = 0.6343$$

Die Figur 19 zeigt die bzgl. Gleichung (2.3) optimierte Dosis-/Wirkungs-Kurve und die Tabelle in Figur 20 die Veränderung der sieben Parameter während der Optimierung. In Figur 21 ist die Entwicklung von $\hat{S}$ bzw. $Z'$ während der Optimierung aufgetragen. Man beachte, dass $S$ hier nicht monoton fällt, da bzgl. $S_{ETAR}$ optimiert wurde.

Anstatt jetzt auch die anderen U2OS-Datensätze analog zu optimieren, wurden die für die erste Mikrotiterplatte optimalen Parameter aus der Tabelle (Figur 20) auf die restlichen drei Datensätze angewandt. Da die Aufnahmen unter vergleichbaren Bedingungen geschehen sind, müssten die Parameter ähnlich gute Dosis-/Wirkungs-Kurven liefern. Es ergeben sich die in Figur 22 aufgeführten Werte.

| | $Z'$ | $\hat{S}$ | $r_o$ | $r_{max}$ | $\log_{10}EC_{50}$ | $s$ |
|---|---|---|---|---|---|---|
| 070116_Platte1 | 0.6343 | $7.13 \cdot 10^{-3}$ | 8.5661 | 70.4336 | 0.9436 | 3.3240 |
| 070116_Platte2 | 0.6825 | $6.16 \cdot 10^{-3}$ | 9.3827 | 714.504 | 1.1505 | 3.1752 |
| 060622_ETAR_Draq | 0.8233 | $240 \ 10^{-3}$ | 3.8020 | 80.0043 | 1.1768 | 3.1063 |

| AFo_U2OS_20xW_070209 | 0.8521 | 4.31·10⁻³ | 4.9235 | 682.637 | 0.9190 | 3.3525 |

Figur 23 zeigt die drei Kurven einzeln mit Fehlerbalken und zum besseren Vergleich zusätzlich alle zusammen in einem Plot.

CHO-Zellen:

Zum Schluss wurde die $\hat{S}$-Optimierung mit Strafterm gemäß Gleichung (2.3) auf die CHO-Zellen angewendet. Die Optimierung geschieht wie bei den U2OS-Zelle in den zwei Stufen. Der Datensatz Afo_070129_CHO_ETAR wurde optimiert und die Parameter dann auf den zweiten Datensatz angewendet. Da sich die Daten der U2OS- und CHO-Zellen ähneln, wurden die Ergebnisse der Tabelle gemäß Figur 20 als Startparameter ausgewählt. Diese liefern die vergleichsweise guten Ergebnisse.

|  | $Z'$ | $\hat{S}$ |
|---|---|---|
| AFo_070129_CHO_ETAR | -0.1793 | 30.77 · 10⁻³ |
| AFo_CHO_ETAR_20x_070221 | -0.1958 | 28.07 · 10⁻³ |

Die CHO-Datensätze sind mit zehn Subwells pro Well größer als die der U2OS Zellen und die Auswertung ist mit 10 Minuten deutlich aufwändiger. Die Optimierung mit der Zielfunktion (2.3) und λ = 0.001 ergab nach insgesamt 330 Funktionsauswertungen und 55 Stunden folgende Ergebnisse

|  | $Z'$ | $\hat{S}$ |
|---|---|---|
| AFo_070129_CHO_ETAR | 0.3041 | 12.41 · 10⁻³ |
| AFo_CHO_ETAR_20x_070221 | 0.3025 | 13.08 · 10⁻³ |

|  | $r_0$ | $r_{max}$ | $Log_{10}EC_{50}$ | $s$ |
|---|---|---|---|---|
| AFo_070129_CHO_ETAR | 24.3209 | 65.0752 | 1.2327 | 4.9058 |
| AFo_CHO8_ETAR_20x_070221 | 19.3795 | 69.2861 | 1.1153 | 11.0819 |

Man beachte, dass hier der Datensatz AFo_070129_CHO_ETAR optimiert wurde und die Ergebnisparameter auf den zweiten Datensatz angewendet wurden. In der Tabelle gemäß Figur 24 sind die sieben optimierten Parameter wiedergegeben und in Figur 25 sind die zugehörigen Dosis-/Wirkungs-Kurven dargestellt. Die Entwicklungen des $\hat{S}$- und des $Z'$-Wertes sind ähnlich wie bei den U2OS-Zellen vorher und auf deren Abbildung wird daher hier verzichtet.

Fazit und Bemerkungen zu den Beispielen.

Die Anwendung der Nelder-Mead-Parameteroptimierung bzgl. des $Z'$-Wertes auf den Akt3-Assay hat eine deutliche Verbesserung hervorgebracht. Besonders die Optimierung des PV-Datensatzes lieferte erstaunliche Ergebnisse und die High-Content-Analyse ist hier erst durch diese optimierten Parameter brauchbar. Dennoch wurde ein entscheidender Nachteil bei der Optimierung bzgl. $Z'$ sichtbar: Da in die Berechnung des $Z'$-Wertes nur die Kontroll-Wells einfließen, kann sich das Residuum der Dosis-/Wirkungs-Regression wahrend der Optimierung verschlechtern.
Die Anwendung der Parameteroptimierung bzgl. des $\hat{S}$-Wertes auf die Akt3-Daten verbesserte die Parametereinstellung ebenfalls und das Residuum der Dosis-/Wirkungs-Regression verringerte sich. Während dieser Optimierung nahm auch der $Z'$-Wert erheblich zu, da in die Berechnung von $\hat{S}$ auch minimale und maximale Dosen einfließen.
Noch beeindruckender als die Parameteroptimierung für den Akt3-Assay ist die Anwendung auf den $ET_AR$-Assay. Durch das Einführen des Strafterms konnte die Minimierungsfunktion an die Eigenarten des Assays angepasst werden. Die Optimierungsroutine erzielte besonders bei den U20S-Zellen eine sehr beträchtliche Verbesserung, obwohl sehr schlechte Startparameter gewählt wurden und keine Informationen über die Funktionsweise des Spot-Detektions-Moduls vorhanden waren. Ebenfalls sehr positiv ist, dass die Ergebnisse der Optimierung bzgl. eines Datensatzes auch auf die anderen Datensatze, die unter ähnlichen Bedingungen aufgezeichnet wurden, anwendbar waren und gute Ergebnisse lieferten.
Die erfindungsgemäße Parameteroptimierung mit Hilfe der Ruckf"uhrung der statistischen Auswertung (siehe Figur 1) ist ein sehr geeignetes Hilfsmittel bei der High-Content-Analyse. Besonders die Optimierung bzgl. des $\hat{S}$-Wertes

scheint sehr geeignet. Dieser stellt sich zwar als etwas unhandlich dar, es hat sich allerdings gezeigt, dass sich bei der $\hat{S}$-Minimierung auch der $Z'$-Wert deutlich verbessert. Die teilweise sehr langen Rechenzeiten wirken auf den ersten Blick etwas abschreckend. Durch eine Parallelisierung der Berechnung der Zielfunktion durch Aufteilen der Daten in Wells oder Subwells und den Einsatz eines leistungsstärkeren Rechners können diese allerdings noch sehr deutlich reduziert werden.

Die Anwendung auf die Beispiel-Assays hat mehrere mögliche Anwendungsgebiete aufgezeigt:

1. Das Optimierungsverfahren kann nach einer Modellierung der Wechselwirkung und groben Voreinstellung der Parameter zur Feineinstellung dieser genutzt werden (siehe Anwendung auf den Whatman-Datensatz des Akt3-Assays).

2. Es eignet sich außerdem für die Einstellung der Parameter von Standard-Bildverarbeitungsroutinen ausgehend von Default-Parametern, auch ohne dass Hintergrundwissen zur Funktionsweise vorhanden ist. Damit ist der Algorithmus für die Parameteroptimierung einer Black-Box nutzbar (siehe Anwendung auf den $ET_A R$-Assay).

3. Die hergeleitete Methode kann weiter angewendet werden, um die Parameter eines bestehenden Modells an andere aber sehr ähnliche Versuchsbedingungen, wie z.B. andere Mikrotiterplatten oder Belichtungszeiten, anzupassen (siehe Anwendung auf den PV-Datensatz des Akt 3-Assays).

**Patentansprüche**

1.  Verfahren zur Analyse der Wirkung einer Testsubstanz auf biologische und/oder biochemische Proben, umfassend die Schritte:

    a. Bereitstellen einer Mehrzahl von Proben im Hochdurchsatz- oder High-Content-Screening, die jeweils eine bekannte Konzentration der Testsubstanz aufweisen, wobei mindestens drei unterschiedliche Konzentrationen der Testsubstanz vorgesehen sind,
    b. Durchführen von Bildaufnahmen, die eine Mehrzahl von Bilddaten je Probe liefern;
    c. Bereitstellen einer Bildverarbeitungsroutine, die aus den Bilddaten einer Probe jeweils einen Aktivitätswert ermittelt, der die Wirkung der Testsubstanz bei der vorliegenden Konzentration beschreibt und wobei die Auswertevorschrift von mindestens einem Steuerparameter beeinflusst wird;
    d. Festlegen mindestens eines Startwerts für den mindestens einen Steuerparameter;
    e. Auswerten der Bilddaten mittels der Auswertevorschrift;
    f. Bestimmen eines Maßes für die Übereinstimmung der ermittelten Aktivitäten mit einem sich aus theoretischen Überlegungen ergebenden funktionalen Modell mit einer die Abhängigkeit der Aktivitäten von der Konzentration beschreibenden Dosis-/Wirkungskurve;
    g. Modifizieren mindestens eines Steuerparameters und Wiederholen der Schritte e. bis g. solange ein Abbruchkriterium nicht erreicht ist.

2.  Verfahren nach Anspruch 1, wobei die biologischen Proben biologische Zellen umfassen, deren Reaktion auf die Testsubstanz untersucht wird.

3.  Verfahren nach einem der vorhergehenden Ansprüche, wobei die Bilddaten mikroskopische Bilddaten umfassen.

4.  Verfahren nach einem der vorhergehenden Ansprüche, bei welchem die Proben mit fluoreszierenden und/ oder lumineszierenden Markern markiert werden.

5.  Verfahren nach einem der vorhergehenden Ansprüche, wobei mehrere Bildaufnahmen je Probe, beispielsweise bei mehreren Anregungs- und/oder Emissionswellenlängen und/oder zu mehreren Zeitpunkten durchgeführt werden.

6.  Verfahren nach einem der vorhergehenden Ansprüche, wobei die Proben zwischen 3 und 30 unterschiedliche Konzentrationen, insbesondere zwischen 5 und 15 unterschiedliche Konzentrationen repräsentieren.

7.  Verfahren nach einem der vorhergehenden Ansprüche, wobei jeweils mehrere Proben mit gleicher Konzentration an Testsubstanz bereitgestellt werden, die den gleichen Punkt der Dosis-/Wirkungskurve repräsentieren.

**8.** Verfahren nach einem der vorhergehenden Ansprüche, wobei jeweils mehrere räumliche Teilbereiche einer Probe aufgenommen werden.

**9.** Verfahren nach einem der vorhergehenden Ansprüche, wobei die Auswertevorschrift Schritte umfasst zur Segmentierung, Objekterkennung, Bestimmung von geometrischen Größen, Helligkeitswerten, Texturparametern von Gesamtbild und/oder im Bild erkannten Segmenten/Objekten und/ oder zur Bestimmung der Anzahl von Objekten, relativer Position von Objekten zueinander, z.B. Spots in Zellen, Relationen von Helligkeiten in unterschiedlichen Messkanälen verschiedener Marker, statistischen Größen, die aus den vorher genannten Größen abgeleitet sind, z. B. Quantile, Prozentsatz von Objekten/ Zellen mit einer bestimmten Eigenschaft, Eigenschaften von Untermengen, d.h. Teilpopulationen, von Objekten.

**10.** Verfahren nach einem der vorhergehenden Ansprüche, wobei die Steuerparameter einen oder mehrere der folgenden umfassen: Helligkeitsschwellen, Größenschwellen, Lage und Größe von Masken zur Integration oder Objektsuche und/ oder Schwellwerte und/ oder Gewichtsfaktoren.

**11.** Verfahren nach einem der vorhergehenden Ansprüche, wobei das funktionale Modell für die Dosis-/Wirkungskurve die Hill-Equation aufweist.

**12.** Verfahren nach einem der vorhergehenden Ansprüche, wobei das Bestimmen des Maßes der Übereinstimmung der ermittelten Aktivitäten mit dem funktionalen Modell für die Dosis-/Wirkungskurve die folgenden Schritte umfasst:

- numerische Anpassung des funktionalen Modells an die ermittelten Aktivitäten und
- Bestimmung einer normierten mittleren quadratischen Abweichung der ermittelten Aktivitäten von dem angepassten funktionalen Modell.

**13.** Verfahren nach einem der vorhergehenden Ansprüche, wobei das Abbruchkriterium eines oder mehrere der folgenden Kriterien umfasst:

- Unterschreiten einer vorher bestimmten normierten mittleren quadratischen Abweichung und/ oder
- Unterschreiten einer vorher bestimmten Mindest-Änderung von Schritt zu Schritt und/oder
- Erreichen einer Maximalanzahl von Iterationsschritten

**14.** Verfahren nach einem der vorhergehenden Ansprüche, wobei das Modifizieren der Steuerparameter nach dem Nelder/Mead Iterationsalgorithmus geschieht.

**15.** Verfahren nach einem der vorhergehenden Ansprüche, wobei in einem mehrstufigen Verfahren zunächst eine erste Gruppe von Steuerparametern optimiert wird bis zum Erreichen eines ersten Abbruchkriteriums, und anschließend eine zweite Gruppe von Steuerparametern optimiert wird bis zum Erreichen eines zweiten Abbruchkriteriums.

**Claims**

**1.** A method for analysing the effect of a test substance on biological and/or biochemical samples, said method comprising the following steps:

a. providing a plurality of samples in high-throughput screening or high-content screening, each comprising a known concentration of the test substance, wherein at least three different concentrations of the test substance are used;
b. taking images which provide a plurality of image data per sample;
c. providing an image processing routine which uses the image data of a sample to determine a respective activity value describing the effect of the test substance at the present concentration, the evaluation rule being influenced by at least one control parameter;
d. determining at least one starting value for the at least one control parameter;
e. evaluating the image data using the evaluation rule;
f. determining the measure of the correspondence between the determined activities and a functional model which results from theoretical considerations and has a dose/effect curve describing the dependence of the activities on the concentration;
g. modifying at least one control parameter and repeating said steps e. to g. as long as an abort criterion has not been reached.

2. The method according to claim 1, wherein the biological samples comprise biological cells whose reaction to the test substance is to be examined.

3. The method according to any one of the preceding claims, wherein the image data comprise microscopic image data.

4. The method according to any one of the preceding claims, wherein the samples are marked by fluorescent and/or luminescent markers.

5. The method according to any one of the preceding claims, wherein a plurality of image recordings are performed per sample, e.g. at a plurality of excitation and/or emission wavelengths and/or at a plurality of times.

6. The method according to any one of the preceding claims, wherein the samples represent from 3 to 30 different concentrations, particularly from 5 to 15 different concentrations.

7. The method according to any one of the preceding claims, wherein, each time, a plurality of samples with the same concentration of the test substance are provided which represent the same point on the dose/effect curve.

8. The method according to any one of the preceding claims, wherein, each time, a plurality of spatial partial regions of a sample are recorded.

9. The method according to any one of the preceding claims, wherein the evaluation rule includes steps for segmentation, object detection, determination of geometric values, brightness values, texture parameters of the total image and/or segments/objects detected in the image, the number of objects, the relative position of objects in relation to each other, e.g. spots in cells, the relation of brightness degrees in different measurement channels of different markers, statistical values derived from the above mentioned values, e.g. quantiles, the percentage of objects/cells having a specific property, properties of subsets, i.e. sub-populations, of objects.

10. The method according to any one of the preceding claims, wherein the control parameters comprise one or a plurality of the following: brightness thresholds, value thresholds, position and size of masks for integration or object search, and/or threshold values and/or weighting factors.

11. The method according to any one of the preceding claims, wherein the functional model for the dose/effect curve comprises the Hill equation.

12. The method according to any one of the preceding claims, wherein the determining of the measure of the coincidence of the detected activities with the functional model for the dose/effect curve comprises the following steps:

   - numerical adaptation of the functional model to the detected activities, and
   - determining a normalized average square deviation of the detected activities from the adapted functional model.

13. The method according to any one of the preceding claims, wherein the abort criterion comprises one or a plurality of the following criteria:

   - falling short of a previously determined normalized average square deviation, and/or
   - falling short of a previously determined minimum change from step to steps, and/or
   - reaching a maximum number of iteration steps.

14. The method according to any one of the preceding claims, wherein the modifying of the control parameters is performed according to the Nelder/Mead iteration algorithm.

15. The method according to any one of the preceding claims, wherein, in a multi-stage method, there is first optimized a first group of control parameters until a first abort criterion has been reached, and, subsequently, a second group of control parameters is optimized until a second abort criterion has been reached.

**Revendications**

1. Procédé d'analyse de l'effet d'une substance test sur des échantillons biologiques et/ou biochimiques, comprenant les étapes suivantes:

   a. préparer plusieurs échantillons par criblage à haut débit ou criblage à contenu élevé, chaque échantillon présentant une concentration connue de la substance test, ou au moins trois concentrations différentes de la substance test sont prévues,
   b. prendre des images qui fournissent plusieurs données d'images par échantillon;
   c. pourvoir une routine de traitement d'images qui détermine une valeur d'activité respectif à partir des données d'images, qui décrit l'effet de la substance test à la concentration présente, et la règle d'évaluation étant influencée par au moins un paramètre de commande;
   d. définir au moins une valeur initiale pour ledit au moins un paramètre de commande;
   e. évaluer les données d'images à l'aide de la règle d'évaluation;
   f. déterminer une mesure de congruence des activités déterminées avec un modèle fonctionnel obtenu par réflexion théorique et comprenant une courbe dose/effet décrivant la dépendance desdites activités de la concentration;
   g. modifier au moins un paramètre de commande et réitérer les étapes e. à g. à moins qu'un critère d'arrêt ne soit pas rempli.

2. Procédé selon la revendication 1, dans lequel les échantillons biologiques comprennent des cellules biologiques dont la réaction sur la substance test est recherchée.

3. Procédé selon l'une des revendications précédentes, dans lequel les données d'images comprennent des données d'images microscopiques.

4. Procédé selon l'une des revendications précédentes, dans lequel les échantillons sont marqués avec des marqueurs fluorescents et/ou luminescents.

5. Procédé selon l'une des revendications précédentes, dans lequel plusieurs images sont prises per échantillon, par exemple à plusieurs longueurs d'onde d'excitation et/ou d'émission et/ou à plusieurs moments.

6. Procédé selon l'une des revendications précédentes, dans lequel les échantillons représentent de 3 à 30 concentrations différentes, notamment de 5 à 15 concentrations différentes.

7. Procédé selon l'une des revendications précédentes, dans lequel plusieurs échantillons avec la même concentration en substance test sont pourvus, respectivement, qui représentent le même point de la courbe dose/effet.

8. Procédé selon l'une des revendications précédentes, dans lequel des images de plusieurs sections spatiales d'un échantillon sont prises, respectivement.

9. Procédé selon l'une des revendications précédentes, dans lequel ladite règle d'évaluation comprend des étapes de segmentation, de détection des objets, de détermination des dimensions géométriques, des valeurs de brillance, des paramètres de texture d'une image totale et/ou de segments/objets détectés dans l'image et/ou de détermination du nombre des objets, de la position relative des objets, par exemple de spots dans des cellules, de relations de brillance dans des canaux de mesurage différents pour différents marqueurs, de valeurs statistiques dérivées des valeurs susmentionnées, par exemple quantiles, pourcentage d'objets/cellules avec une certaine caractéristique, caractéristiques de sous-ensembles, soit populations partielles, d'objets.

10. Procédé selon l'une des revendications précédentes, dans lequel lesdits paramètres de commande comprennent un ou plusieurs des paramètres suivants: seuils de brillance, seuils de dimension, positions et dimensions de masques pour l'intégration ou la recherche des objets et/ou valeurs de seuils et/ou facteurs de poids.

11. Procédé selon l'une des revendications précédentes, dans lequel ledit modèle fonctionnel pour la courbe de dose/effet comprend une équation de Hill.

12. Procédé selon l'une des revendications précédentes, dans lequel la détermination de la dégrée de congruence des activités déterminées avec ledit modèle fonctionnel pour la courbe dose/effet comprend les étapes suivantes:

- l'adaptation numérique du modèle fonctionnel aux activités déterminées, et
- la détermination d'un écart quadratique moyen normé des activités déterminées dudit modèle fonctionnel adapté.

**13.** Procédé selon l'une des revendications précédentes, dans lequel ledit critère d'arrêt comprend un ou plusieurs des critères suivants:

- rester inférieur à écart quadratique moyen normé prédéterminé et/ou
- rester inférieur à une variation minimale prédéterminée d'un pas à l'autre, et/ou
- atteindre un nombre maximal de pas d'itération.

**14.** Procédé selon l'une des revendications précédentes, dans lequel la modification des paramètres de commande est faite conformément à algorithme d'itération de Nelder/Mead.

**15.** Procédé selon l'une des revendications précédentes, dans lequel dans un procédé à plusieurs étapes un premier groupe de paramètres de commande est optimisé d'abord jusqu'à ce qu'un premier critère d'arrêt soit atteint, et ensuite un deuxième groupe de paramètres de commande est optimisé jusqu'à ce qu'un deuxième critère d'arrêt soit atteint.

EP 2 128 815 B1

Fig.1

Fig.2

EP 2 128 815 B1

Histogram Membrane, stimulated Cell

(a)

Fig.3

Histogram Membrane, unstimulated Cell

(b)

Histogram Cytoplasm, stimulated Cell

(a)

Fig.4

Histogram Cytoplasm, unstimulated Cell

(b)

23

Fig.5

Fig.6

Fig.7

$$q_1 = 0.8 \longmapsto 0.6485$$
$$q_2 = 0.9 \longmapsto 1.0$$
$$\mu_1 = 1.0 \longmapsto 0.46363$$
$$\mu_2 = 1.0 \longmapsto 0.45558$$
$$\lambda = 1.0 \longmapsto 1.2098$$

Zone 1:
$$0 \longmapsto 2.5542$$
$$4 \longmapsto 3.6914$$

Zone 2:
$$7 \longmapsto 4.7645$$
$$13 \longmapsto 11.9581$$

**Fig.8**

**Fig.9**

(a)

(b)

(c)

(d)

**Fig.10**

Fig.11

Fig.12

$$q_1 = 0.8 \mapsto 0.13467$$
$$q_2 = 0.9 \mapsto 0.43262$$
$$\mu_1 = 1.0 \mapsto 0.28786$$
$$\mu_2 = 1.0 \mapsto 0.24944$$
$$\lambda = 1.0 \mapsto 0.99737$$

Zone 1:
$$0 \mapsto 0.048674$$
$$4 \mapsto 3.9627$$

Zone 2:
$$7 \mapsto 7.0055$$
$$13 \mapsto 12.2013$$

Fig.13

Fig.14

(a)

(b)

(c)

(d)

Fig.15

Fig.16

Fig.17

Fig.18

Fig.19

Zone 1:
  -5.0   ↦   -8.0685
Zone 2:
  5.0   ↦   9.4430
SpotMinimumDistance:
  3.0   ↦   16.6058
SpotPeakRadius:
  0.0   ↦   1.9822
SpotReferenceRadius:
  3.0   ↦   61.9845
SpotMinimumContrast:
  0.3   ↦   0.4448
SpotMinimumToCellIntensity:
  1.0   ↦   0.0

Fig.20

$$\widehat{S} \qquad\qquad Z'$$

Fig.21

|  | $Z'$ | $\widehat{S}$ | $r_0$ | $r_{\max}$ | $\log_{10} EC_{50}$ | $s$ |
|---|---|---|---|---|---|---|
| 070116_Platte1 | 0.6343 | $7.13 \cdot 10^{-3}$ | 8.5661 | 70.4336 | 0.9436 | 3.3240 |
| 070116_Platte2 | 0.6825 | $6.16 \cdot 10^{-3}$ | 9.3827 | 71.4504 | 1.1505 | 3.1752 |
| 060622_ETAR_Draq | 0.8233 | $2.40 \cdot 10^{-3}$ | 3.8020 | 80.0043 | 1.1768 | 3.1063 |
| AFo_U2OS_20xW_070209 | 0.8521 | $4.31 \cdot 10^{-3}$ | 4.9235 | 68.2637 | 0.9190 | 3.3525 |

Fig.22

070116_Platte2

060622_ETAR_Draq                    AFo_U2OS_20xW_070209

Fig.23

29

Zone 1:
    -5.0   ↦   -19.4999

Zone 2:
    5.0   ↦   19.5236

SpotMinimumDistance:
    3.0   ↦   11.5618

SpotPeakRadius:
    0.0   ↦   3.0686

SpotReferenceRadius:
    3.0   ↦   64.6338

SpotMinimumContrast:
    0.3   ↦   0.2615

SpotMinimumToCellIntensity:
    1.0   ↦   0.4173

Fig.24

Fig.25

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

• WO 2006063216 A

**In der Beschreibung aufgeführte Nicht-Patentliteratur**

• **Alt, W.** Nichtlineare Optimierung. Vieweg, 2002

• **Kelley, C.T.** Iterative Methods for Optimization. Society for Industrial & Applied Mathematics, 1999